# EUROPEAN PATENT APPLICATION

(11) **EP 1 607 006 A1**
(43) Date of publication of application: **21.12.2005**
(21) Application number: 04076795.6
(22) Date of filing: 18.06.2004
(51) Int. Cl.: A23L 2/02, A61K 35/78, A23L 1/30, A61P 9/00

(54) **Functional berry composition**

(71) Applicant: UNILEVER N.V., 3013 AL Rotterdam (NL)
(72) Inventor: Draijer, Richard, 3133 AT Vlaardingen (NL); Louter, Adrianus Johannes Harmannus, 3133 AT Vlaardingen (NL); Rechner, Andreas Roland, 35043 Marburg (DE)
(74) Representative: Kleiborn, Paul Erik

(57) **Abstract**

The invention relates to a food composition comprising elderberry and/or blackcurrant, wherein the food composition has a polyphenol content of 300 mg/kg or more, calculated as gallic acid equivalents, wherein the consumption of the food composition by a human subject is effective in reducing the cardiovascular risk of that human subject.

## Description

### Field of the invention

The invention relates to functional berry compositions, i.e. food compositions comprising one or more berries as ingredient, having a health effect in humans.

### Background to the invention

Health is one of the major concerns of consumers. Surveys indicate the consumer wants to be free from health problems and stay healthy for longer. That food plays a key role in maintaining a healthy body and mind is well accepted. One of the major health concerns is the cardiovascular system. This system depends on a well-balanced interaction between the heart, the blood and the blood vessels (the vasculature).

There are some proposals for commercial and non-commercial functional food products that are suitable for reducing the risks; in particular related to reducing of LDL-cholesterol levels and reduction of blood pressure. High LDL-cholesterol levels and high blood pressure are considered major risk factors for cardiovascular disease.

Another major risk factor is blood clotting and blocking of arteries. These risks are related to the condition of the blood vessels and blood, more particular to the platelet function and endothelia function. Therefore there is a need for functional food products that are suitable for keeping the blood and blood vessels in good condition.

There is a considerable interest in the health effects of polyphenols. One group of polyphenols studied for their health effects is the group of anthocyanins. Anthocyanins are a class of polyphenols and natural colorants responsible for the red to purple colour of many berries, fruits, vegetables and flowers. The berries mentioned herein are collectively referred to as red berries. The naturally occurring anthocyanins are the glycosides of the anthocyanidins. Their colour is pH-dependent showing a deep red to purple at low pH (< 5), a light yellow or no colour with increasing pH (pH 5-6), and a deep purple to blue colour at basic pH (pH > 7). The chemical structure is shown in figure 1.

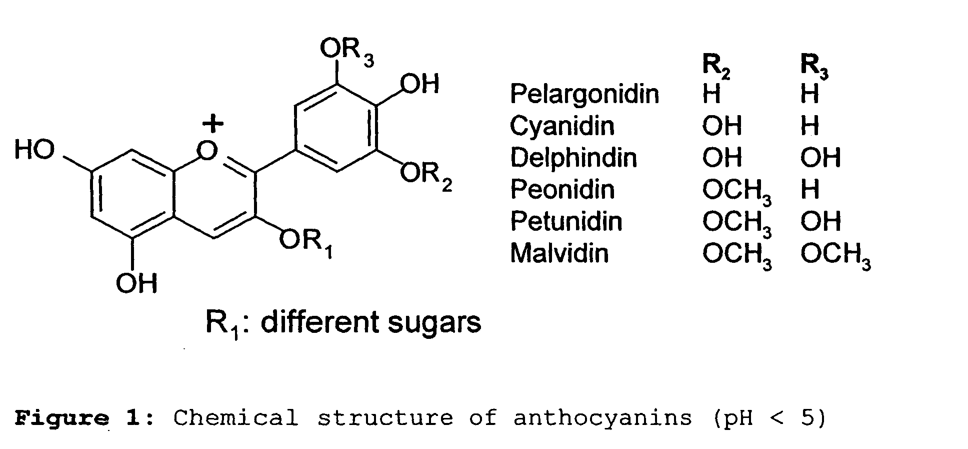

Berries are particularly rich in anthocyanins, and berries are known to show antioxidant properties. Anthocyanins are often the major contributors to the antioxidant capacities detected in red berries (Rechner, 2001). Elderberry anthocyanins have also been shown to protect endothelial cells against oxidative stress (Youdim et al., 2000). The anthocyanin aglycone delphinidin (in red grapes, blueberries and blackcurrants) has been reported to cause an endothelium-dependent vasorelaxation, at least in rats (Andriambeloson et al., 1998) and to inhibit angiogenesis *in vitro* and *in vivo* (Favot et al., 2003). Anthocyanin-rich extracts from maize have been shown to suppress the incidence and number of colorectal adenomas and carcinomas induced in rats (F-344) by a carcinogenic maillard product (Hagiwara et al., 2001).

The anti-bacterial properties of anthocyanins were tested in agar diffusion experiments showing growth inhibition of mutant E. coli strain CM 871 but no inhibitory effect on the other test bacteria (Puupponen-Pimiä et al., 2001). The data collection above shows the wide range of biological effects anthocyanins are accounted for and thus, their potential as powerful health ingredients.

Red berries are an excellent source for anthocyanins but also other polyphenols such as procyanidins, flavanols, flavonols, hydroxycinnamates and ellagitannins.

### Summary of the invention

It is therefore an object of the invention to provide a food composition having an effect in human subject of decreased risk of disorders of the blood vessels and blood. Another object is to provide such food composition having an acceptable taste, preferably a good taste. One or more of these objects is attained in the food composition according to the invention comprising elderberry and/or blackcurrant, wherein the food composition has a polyphenol content of 300 mg/kg or more, calculated as gallic acid equivalents, wherein the consumption of the food composition by a human subject is effective in reducing the cardiovascular risk of that human subject.

The invention further relates to a liquid food composition being a mixture of grape juice and/or apple juice and berry juice, having a polyphenol content of 300 mg/kg or more, calculated as gallic acid equivalents, for use as a medicament or as a physiologically functional food product.

### Detailed description of the invention

The polyphenol content of the food compositions according to the invention should be 300 mg/kg or more, calculated as gallic acid equivalents. If the polyphenol content is lower than 300 mg/kg little or no effect on reducing cardiovascular risk will be obtained. The polyphenol content of a food product may be measured as described herein in the examples. Preferably the food composition has a polyphenol content of 1000-20000 mg/kg.

Preferably, the food composition is such that the consumption of the food composition by a human subject is effective in causing in the human subject one or more, preferably two or more, more preferably three or more, even more preferably four, and most preferably all five of the following effects:
a) reduction of hypertension
b) inhibition of platelet aggregation
c) reduction of level of total cholesterol
d) reduction of level of LDL cholesterol
e) improvement of endothelial function

Preferably, wherein the food composition has a polyphenol content of 1000-20000 mg/kg.

Advantageously, the food composition comprises 100 mg/kg or more of vitamin C. The berry containing food compositions according to the invention will have a natural content of vitamin C, because vitamin C is present in many berries at a relatively high level.

Advantageously, the elderberry and/or blackcurrant are present in the food composition as juice or extract. An extract is especially preferred, since the use of extract gives more flexibility for food product design because the amount of polyphenols per volume unit is high.

Preferably, in addition to the elderberry (Sambucus nigra) and/or blackcurrant (Ribes nigrum L.), the food composition according to the invention comprises one or more of blueberry (*Vaccinium corymbosum, Vaccinium ashei or Vaccinium angustifolium*), bilberry *(Vaccinium myrtillus*), blackberry *(Rubus fructicosus*), red raspberry (*Rubus idaeus*), cranberry (*Vaccinium macrocarpon*) and black chokeberry (*Aronia melanocarpa*).

The total polyphenol content of berry juices used according to the invention is given in table 1.

**Table 1:**

| Antioxidant capacity (TEAC), total polyphenols and anthocyanin content of selected fruit juices (n > 3) from Rechner 2001 | | | |
|---|---|---|---|
| | **TEAC** [mmol/L Trolox] | **Total polyphenols** [mg/L catechin] | **Total anthocyanins** [mg/L cyanidin-3-glucoside] |
| **Elderberry** | 55.0 | 9955 | 6068 |
| **Blackcurrant** | 32.4 | 5870 | 3339 |
| **Blackberry** | 12.2 | 3665 | 1477 |
| **Sour cherry** | 23.2 | 4821 | 1019 |
| **Red currant** | 8.7 | 1815 | 277 |
| **Strawberry** | 14.2 | 2111 | 117 |

The invention further related to a liquid food composition being a mixture of grape juice and berry juice, for use as a medicament or as a physiologically functional food product. The mixture of grape juice and berry juice has a good taste, since the grape juice has a high natural sugar content. Further the food composition has a high polyphenol content, at least 300mg/kg, calculated as gallic acid equivalents. The polyphenols in the liquid food composition have a broad spectrum since the polyphenols originate from berry as well as from grape. Preferably the grape juice is obtained from red grapes.

The liquid food composition is preferably such that the consumption of the food composition by a human subject is effective in causing in a human subject in causing in a human subject is effective in reducing the cardiovascular risk of that human subject.

More preferably the food composition is effective in causing in the human subject one or more, preferably two or more, more preferably three or more, and most preferably all four of the following effects:
a) reduction of hypertension
b) inhibition of platelet aggregation
c) reduction of level of total cholesterol
d) reduction of level of LDL cholesterol
e) improvement of endothelial function

Preferably the berry juice is blackcurrant (*Ribes nigrum L.*) juice and/or elderberry (Sambucus Nigra) juice.

The food compositions according to the invention are characterised by the origin of the ingredients (berry and grape origin). In a food composition the origin of the ingredients may be determined by analysis of the polyphenols in the food products. Although red berries and red grape are quite similar concerning their content of polyphenol subgroups (anthocyanins, flavonols, flavanols, hydroxycinnamates, procyaninds), on an individual compound level their composition differs significantly. Most red berries contain specific polyphenols often exclusive for one specific berry. Table 2 lists the most prominent compounds specific for some red berries. Anthocyanin fingerprinting by HPLC may be used to characterise the different berries.

**Table 2:**

| Typical berry polyphenols for characterisation | |
|---|---|
| **Berry** | **Characteristic component** |
| Elderberry | Cyanidin-3-sambubioside (anthocyanin) |
| Blackcurrant | delphinidin-3-rutinoside (anthocyanin) |
| Blueberry | delphindin-3-galactoside or malvidin-3-galactoside (anthocyanins) |
| Bilberry | delphindin-3-galactoside or malvidin-3-galactoside (anthocyanins) |
| Blackberry | cyanidin-3-glucoside (anthocyanin) |
| Raspberry | ellagic acid |
| Cranberry | quercetin-3-galactoside (flavonol), peonidin-3-galactoside, (benzoic acid) |
| Black chokeberry | Cyanidin-3-galactoside (anthocyanin) |

The amount of polyphenols in the food composition may be any amount that is effective in a health effect in a human, when consumed in an ordinary way. Preferably, the amount of polyphenols is the food composition is such that the amount of polyphenols in an average serving of the food composition is 150 mg or more, more preferably 300 mg or more, even more preferably 500 mg or more and most preferably 1000-2000 mg. The average serving size for a food product according to the invention may be indicated herein. Alternatively, for food products of which the average serving size is not given herein, average serving size is defined as the "reference amounts of food customarily eaten at one time" found in the Food Nutrition Tables of the Food and Drug Administration (USA).

The food products according to the invention may be of any food type. They may comprise common food ingredients in addition to the berries, such as flavour, sugar, fruits, minerals, vitamins, stabilisers, thickeners, etc. in appropriate amounts. Preferably the food products are fruit juice products, beverages, bakery products, dairy type products, frozen confectionery products or nutritional bars. These preferred types of food products are described in some detail below and in the examples. These preferred food products may advantageously be used as part of a fat containing meal, or be used in conjunction with such a meal.

Examples of food products according to the invention are:

### • Fruit juice products

Examples of fruit juice products according to the invention are juices derived from citrus fruit like orange and grapefruit, tropical fruits, banana, peach, peer, strawberry, in which a a red grape juice or red grape extract and a berry juice or berry extract is mixed, dissolved or suspended. The average serving size for the fruit juice products is 250 ml.

### • Dairy type products

Examples of dairy products according to the invention are milk, dairy spreads, cream cheese, milk type drinks and yogurt, wherein the grape and berry (juice or extract) is mixed, dissolved or suspended. Optionally flavor or other additives may be added.

An example of a composition for a yoghurt type product is about 50-80 wt.% water, 0.1-15 wt.% berry extract, 0.1-15 wt.% grape extract, 0-15 wt.% whey powder, 0-15 wt.% sugar (e.g. sucrose), 0.01-1 wt.% yoghurt culture, 0-20 wt.% fruit, 0.05-5 wt.% vitamins and minerals, 0-2 wt.% flavour, 0-5 wt.% stabiliser (thickener or gelling agent), wherein the berry extract is dissolved or suspended.

A average serving size for a yoghurt type product could be from 50 to 250 g, generally from 80 to 200 g.

### • Frozen Confectionery Products

For the purpose of the invention the term frozen confectionery product includes milk containing frozen confections such as ice-cream, frozen yoghurt, sherbet, sorbet, ice milk and frozen custard, water-ices, granitas and frozen fruit purees.

Preferably the level of solids in the frozen confection e.g. sugar, fat, flavouring etc) is more than 3 wt.%, more preferred from 10 to 70 wt.%, for example 40 to 70 wt.%.

Ice cream will typically comprise 0 to 20 wt.% of fat, 0.1 to 20 wt.% hydrolysed casein solids, sweeteners, 0 to 10 wt.% of non-fat milk components and optional components such as emulsifiers, stabilisers, preservatives, flavouring ingredients, vitamins, minerals, etc, the balance being water. Typically ice cream will be aerated e.g. to an overrun of 20 to 400 %, more specific 40 to 200 % and frozen to a temperature of from -2 to -200 °C, more specific -10 to -30 °C. Ice cream normally comprises calcium at a level of about 0.1 wt%, wherein the berry and or grape (juice or extract) is mixed, dissolved or suspended.

The average serving size of a frozen confectionery product is 85g.

### • Nutrition bars

As example a composition of a granola bar is given. Granola bars consists of a binder syrup which binds the granola and fruit ingredients into a solid bar. E.g. nuts,pieces of chocolate, or a low moisture yoghurt filling can be added as well. The bar can be enrobed with a coating of e.g. chocolate or a low moisture yoghurt layer.

### Granola bar example (weight per cent)

0-40 % granola base (oat flakes, wheat flakes, rice crispies)
0-60 % binder syrup (corn syrup, sugar, salt, lecithine, vegetable oil)
0-40 % fruit filling (low moisture jelly, dried fruits)

Moisture content is typically below 15%.

### More detailed composition:

Oat flakes/wheat flakes 0-25%
Rice crispies 0-15%
Corn syrup 0-50%
Sugar 0-15 %
Lecithine 0-1 %
Salt 0-1%
Vegetable oil 0-25%
Dried fruits 0-20 %
Low moisture fruit jelly 0-20%
Stabilisers 0-2%

Stabilisers are often added to create a more solid fruit filling.

### Optional:

Grape juice extract+wine extract 0-5% to reach 200 mg of polyphenols
Berry extract 0-5% to reach 200 mg of polyphenols
Flavours 0-2%
Vitamins/minerals 0-5%

Products are ambient stable.

### • Food emulsions, (fruit) spreads

The food product may be an oil and water containing emulsion, for instance a spread. Oil and water emulsion is herein defined as an emulsion comprising oil and water and includes oil in water (O/W) emulsions and water in oil emulsions (W/O) and more complex emulsions for instance water-in-oil-in-water (W/O/W/O/W) emulsions. Oil is herein defined as including fat.

Preferably the food product is a spread, frozen confection, or sauce. Preferably a spread according to the invention comprises 30-90 wt.% vegetable oil. Advantageously a spread has a pH of 4.2-6.0.

Other food product according to the invention can be prepared by the skilled person based on common general knowledge, using the red grape (juice or extract) and berry extract as ingredient in suitable amounts. Examples of such food products are baked goods, dairy type foods, snacks, etc.

### Examples

### Determination of polyphenol content

Total polyphenol content was measured using Folin-Ciocalteau colour reagent at mildly basic pH in an UV-spectroscopic method. Quantification was achieved by determining the total polyphenol content as Gallic Acid Equivalents (GAE) or epicatechin equivalents (ECE), using standard curves for gallic acid and epicatechin.

Liquid samples were diluted if the polyphenol content exceeded 1000 mg/L. Interfering L-ascorbic acid or sulphur dioxide were removed with hydrogen peroxide (10µL of 30% hydrogenperoxide for 5 mL sample).

Defined amounts of plant material, extracts, food products, fruit or vegetables were extracted with solvent (e.g. water or alcohol). For dissolving extracts the most suitable solvent (i.e which dissolved the extract without residue). 1-20 mg of extract was dissolved in 1 mL of solvent.

### Measurement:

8.4 mL demineralised water (for blank 8.5 mL)
+ 0.1 mL sample of standard solution
+0.5 mL Folin-Ciocalteu reagent
mix and leave for 5 min
+1.0 mL saturated sodium carbonate solution
mix and leave for 60 min

Following the transfer of a 200 µL aliquot of the samples and the blind into a 96 wells plate (2 wells per sample) the extinction was measured at 720 nm. A standard curve is measured with each measurement and used to calculate the total polyphenol content relative to the standard compound.

### Examples 1-6

Six different blends of juices containing blackcurrant juice and grape or apple juice were prepared having the composition as given in table 3.

### Preparation:

Refrigerated juices were allowed to heat to ambient temperature (22°C). Juices were put in a metal container and mixed with a Silverson high shear mixer during 1 min. Grape juice extract and wine extract were dry blended and slowly added to the juice mixture under stirring (1-2 min). Resulting product was stored at 5 °C.

### Results:

A taste panel evaluated the taste of the products of examples 1-6. The products were scored on a scale from 1 to 10 for sweetness, sourness and astringency. 1 = low/weak in perception, 10 = high/strong in perception. The results are shown in table 3.

**Table 3:**

| Examples 1-6, composition of juice mixes and results of polyphenol analysis and results of tasting. | | | | | |
|---|---|---|---|---|---|
| Product | Sweetness | Sourness | Astringency | PP* (GEA) mg/L | Comments |
| Example 1 BC/AP 29:71 | 7.0 | 3.3 | 3 | 1330 | ++ |
| Example 2 BC/AP 40:60 | 5.0 | 5.7 | 2.5 | 1850 | + |
| Example 3 BC/RG 29:71 | 6.8 | 4.5 | 3 | 1330 | ++ |
| Example 4 BC/RG 40:60 | 6.5 | 4.8 | 2.5 | 1850 | ++, favorite , best |
| Example 5 BC/AP/RG 33:33:33 | 4.8 | 6 | 3.7 | 1520 | + |
| Example 6 BC:AP:RG 43:29:29 | 5.7 | 5.3 | 2.3 | 2000 | + |
| BC = blackcurrant juice from Naturfood, NL (4600 mg/l polyphenols GAE) | | | | | |
| AP = standard commercial apple juice (Appelsientje, Netherlands) | | | | | |
| RG = standard commercial red grape juice (Appelsientje, Netherlands) | | | | | |
| PP* = polyphenols from BC juice | | | | | |
| GEA = gallic acid equivalent | | | | | |

All products were received well, the blend of blackcurrant and red grape (40:60) was percieved as the best by 3 out of the six panel members. Non of the products were received as to sour of astringent. For a 200 mg PP serving a volume of approx. 100 ml is then required. Commercial red grape juice does mask the strong taste of BC juice better then apple juice, which can be attributed to the difference in sugar content, RG juice has 60 % more sugar as AP juice.

### References

1. Rechner AR. (2001) Einfluss der Verarbeitungstechnik auf die Polyphenole und antioxidative Kapazität von Apfel- und Beerenobstsäften. Ph.D. thesis Justus Liebig Universität Giessen (http://bibd.unigiessen.de/ghtm/2001/uni/d010023.htm).
2. Youdim, K. A., Martin, A. & Joseph, J. A. (2000) Incorporation of the elderberry anthocyanins by endothelial cells increases protection against oxidative stress. Free Rad. Biol. Med. 29: 51-60
3. Andriambeloson, E., Magnier, C., Haan-Archipoff, G., Lobstein, A., Anton, R., Beretz, A., Stoclet, J. C. & Andriantsitohaina, R. (1998) Natural dietary polyphenolic compounds cause endothelium-dependent vasorelaxation in rat thoracic aorta. J Nutr. 128: 2324-2333.
4. Favot, L., Martin, S., Keravis, T., Andriantsitohaina, R. & Lugnier, C. (2003) Involvement of cyclin-dependent pathway in the inhibitory effect of delphinidin on angiogenesis. Cardiovasc. Res. 59: 479-487
5. Hagiwara, A., Miyashita, K., Nakanishi, T., Sano, M., Tamano, S., Kadota, T., Koda, T., Nakamura, M., Imaida, K., Ito, N. & Shirai, T. (2001) Pronounced inhibition by a natural anthocyanin, purple corn color, of 2-amino-1-methyl-6-phenylimidazo[4,5-b]pyridine (PhIP)-associated colorectal carcinogenesis in male F344 rats pretreated with 1,2-dimethylhydrazine. Cancer. Lett. 171: 17-25
6. Puupponen-Pimia R, Nohynek L, Meier C, Kahkonen M, Heinonen M, Hopia A, Oksman-Caldentey KM. Antimicrobial properties of phenolic compounds from berries. J Appl Microbiol. 2001 Apr;90(4) :494-507.

## Claims

1. Food composition comprising elderberry and/or blackcurrant, wherein the food composition has a polyphenol content of 300 mg/kg or more, calculated as gallic acid equivalents, wherein the consumption of the food composition by a human subject is effective in reducing the cardiovascular risk of that human subject.

2. Food composition according to claim 1, wherein the consumption of the food composition by a human subject is effective in causing in the human subject one or more, preferably two or more, more preferably three or more, even more preferably four, and most preferably all five of the following effects:
a) reduction of hypertension
b) inhibition of platelet aggregation
c) reduction of level of total cholesterol
d) reduction of level of LDL cholesterol
e) improvement of endothelial function

3. Food composition according to claim 1, wherein the food composition has a polyphenol content of 1000-20000 mg/kg.

4. Food composition according to claim 1 or 2, wherein the food composition comprises 100 mg/kg or more of vitamin C.

5. Food composition according to any of claims 1-3, wherein the elderberry and/or blackcurrant are present in the food composition as juice or extract.

6. Food composition according to claim 4, wherein the elderberry and/or blackcurrant are present in the food composition as extract.

7. Food composition according to any of claims 1-5, wherein the food composition comprises or more of blueberry (*Vaccinium corymbosum, Vaccinium ashei or Vaccinium angustifolium*), bilberry *(Vaccinium myrtillus)*, blackberry (*Rubus fructicosus*), red raspberry (*Rubus idaeus*), cranberry (*Vaccinium macrocarpon*) and black chokeberry (*Aronia melanocarpa*).

8. Liquid food composition being a mixture of grape juice and/or apple juice and berry juice, having a polyphenol content of 300 mg/kg or more, calculated as gallic acid equivalents, for use as a medicament or as a physiologically functional food product.

9. Liquid food composition according to claim 8, wherein the consumption of the food composition by a human subject is effective in reducing the cardiovascular risk of that human subject.

10. Liquid food composition according to claim 9, wherein consumption of the food composition by a human subject is effective in causing in the human subject one or more, preferably two or more, more preferably three or more, even more preferably four, and most preferably all five of the following effects:
f) reduction of hypertension
g) inhibition of platelet aggregation
h) reduction of level of total cholesterol
i) reduction of level of LDL cholesterol
j) improvement of endothelial function

11. Liquid food composition according to any of claims 8 to 10 wherein the grape juice is juice obtained from red grapes.

12. Liquid food composition according to any of claims 8 to 12, wherein the berry juice is blackcurrant (*Ribes nigrum L.*) juice and/or elderberry (Sambucus Nigra) juice.
